# EUROPEAN PATENT SPECIFICATION

(11) **EP 1 937 315 B1**
(45) Date of publication and mention of the grant of the patent: **09.06.2010**
(21) Application number: 06779156.6
(22) Date of filing: 17.08.2006
(51) Int. Cl.: A61K 49/18

(54) **IMAGING AGENT COMPRISING IRON CONTAINING COLLOIDAL PARTICLE CONJUGATED TO OLIGOSACCHARIDES**
BILDGEBENDES MITTEL MIT EISEN, DAS AN OLIGOSACCHARIDE KONJUGIERTE KOLLOIDALE TEILCHEN ENTHÄLT
AGENT D'IMAGERIE RENFERMANT DES PARTICULES COLLOIDALES COMPRENANT DU FER ET CONJUGUEES A DES OLIGOSACCHARIDES

(30) Priority: 19.08.2005 GB 0517077
(43) Date of publication of application: 02.07.2008
(73) Proprietor: ISIS INNOVATION LIMITED, Summertown, Oxford OX2 7SG (GB)
(72) Inventor: DAVIS, Benjamin, Guy, Oxford OX1 3TA (GB); VAN KASTEREN, Sander, Izaak, Oxford OX1 3TA (GB); ANTHONY, Daniel, Oxford OX1 3QT (GB); SIBSON, Nicola, Oxford OX1 3PT (GB)
(74) Representative: Roques, Sarah Elizabeth
(86) International application number: PCT/GB2006/003084
(87) International publication number: WO 2007/020450

(56) References cited:
- WO-A-00/08038
- WO-A-02/46241
- US-A1- 5 352 432
- RYE C S ET AL: "The synthesis of a novel thio-linked disaccharide of chondroitin as a potential inhibitor of polysaccharide lyases" CARBOHYDRATE RESEARCH, ELSEVIER SCIENTIFIC PUBLISHING COMPANY. AMSTERDAM, NL, vol. 339, no. 3, 25 February 2004 (2004-02-25), pages 699-703, XP004487757 ISSN: 0008-6215
- EISELE T ET AL: "Synthesis of a thio-linked Lewis A (Le<a>) epitope" CARBOHYDRATE RESEARCH, ELSEVIER SCIENTIFIC PUBLISHING COMPANY. AMSTERDAM, NL, vol. 306, no. 1-2, January 1998 (1998-01), pages 81-91, XP004123557 ISSN: 0008-6215
- BOCK K; DEFAYE J; DRIGUEZ H; BAR-GUILLOUX E: "CONFORMATIONS IN SOLUTION OF ALPHA,ALPHA-TREHALOSE ALPHA-D-GLUCOPYRANOSYL-ALPHA-D-MANNOPYRANO SIDE AND THEIR 1 THIOGLYCOSYL ANALOGS AND A TENTATIVE CORRELATION OF THEIR BEHAVIOR WITH RESPECT TO THE ENZYME TREHALASE" EUROPEAN JOURNAL OF BIOCHEMISTRY, vol. 131, no. 3, 1983, pages 595-600, XP002424138 ISSN: 0014-2956

## Description

### FIELD OF THE INVENTION

This invention relates to imaging agents and in particular to iron oxide particles having attached thereto sugar moieties and in particular di, tri and higher saccharides. The agents of the present invention can be used as contrast agents, for example in magnetic resonance imaging, and may be used to target receptors such as selectins. The agents can be used in methods of diagnosis, for example to monitor inflammation and in the diagnosis of multiple sclerosis.

### DESCRIPTION OF THE PRIOR ART

A variety of imaging techniques are available for diagnostic purposes. Such imaging techniques are generally non invasive and include magnetic resonance imaging (MRI) and ultrasound. Contrast agents are used in imaging to increase the signal difference between the area of interest and background. Such agents can be divided into two general categories, those which non-specifically enhance the signal that is produced and targeted contrast agents which are modified in order to localise to a specific cell type or tissue through a passive or active mechanism.

One type of contrast agent that is used is super magnetic particles. These particles are iron containing colloidal particles, made of iron oxides and hydroxides. Another agent that is used is gadolinium based agents, such as gadolinium-diethylenetriamine-pentacetic acid (Gd-DTPA). Gd based systems can only infiltrate the brain if there is a breakdown in the blood-brain barrier. Other contrast agents include nanoparticles containing a variety of different agents and microbubbles, that is low-density gas-filled particles in the micron range. A variety of such contrast agents are described in Morawski et al, Current Opinion in Biotechnology 2005 16, 89-92.

There is a need to provide new contrast agents and in particular those which can be targeted to cell surface receptors to enhance imaging methods.

### SUMMARY OF THE INVENTION

The present invention provides a conjugate comprising an iron containing colloidal particle, the particle being conjugated to one or more targeting moieties. The targeting moieties are oligosaccharides which include a saccharide unit selected from GalNAc (N-acetyl galactosamine), GalUA (galacturonic acid), GlcNAc (N-acetyl glucosamine), GlcUA (glucuronic acid), 1dUA (iduronic acid) and a sialic acid. The invention further provides a formulation comprising a conjugate as defined above and a pharmaceutically acceptable excipient.

The invention further provides a process for preparing an intermediate which is useful in the preparation of a conjugate as defined above and which comprises:
(a) providing a compound of formula (I):

   R¹-S-CH₂CN (I)

   wherein R¹ is an acetylated carbohydrate-based moiety;
(b) selectively deprotecting the compound of formula (I) in order to convert the acetylated groups to hydroxy groups;
(c) reacting the product of step (b) with a carbohydrate processing enzyme in order to extend the compound of formula (I) by the addition of one or more carbohydrate-based moieties R²; and
(d) activating the product of step (c) in order to produce a compound of formula (II):

   (R²)ₙ-R¹-S-CH₂C(NH)-O-Me (II)

   wherein n is the number of carbohydrate-based moieties R² added in step (c) and is an integer of from 1 to 10.

Other aspects of the invention include a disaccharide of formula (III):

R²-R¹-S-CH₂-C(NH)-O-Me (III)

wherein R¹ and R² are each monosaccharide moieties.

There is further provided a process for preparing a conjugate comprising an iron-containing colloidal particle, the particle being conjugated to one or more targeting moieties, which targeting moieties are oligosaccharides which process comprises reacting an intermediate of formula (II) or formula (III) as defined above with an iron containing colloidal particle.

### BRIEF DESCRIPTION OF THE DRAWINGS

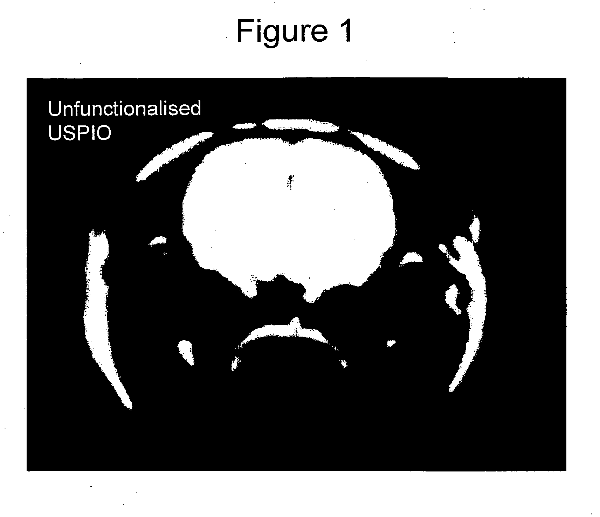

Figures 1 to 3 show MRI scans using two controls (a Gd control and an unconjugated superparamagnetic particle control) and one sample according to the invention which employs a Sialyl Lewis X-conjugated iron containing colloidal particle.

### DETAILED DESCRIPTION OF THE INVENTION

As used herein, an alkyl group or moiety is a linear or branched alkyl group or moiety preferably containing from 1 to 6 carbon atoms such as a C₁₋₄ alkyl group or moiety. Examples of C₁₋₄ alkyl groups and moieties include methyl, ethyl, *n*-propyl, *i-*propyl, *n*-butyl, *i*-butyl and *t*-butyl. For the avoidance of doubt, where two alkyl moieties are present in a group, the alkyl moieties may be the same or different

As used herein the term amino represents a group of formula -NH₂. The term C₁₋₆ alkylamino represents a group of formula -NHR' wherein R' is a C₁₋₆ alkyl group, preferably a C₁₋₄ alkyl group, as defined previously. The term di(C₁₋₆ alkyl)amino represents a group of formula -NR'R" wherein R' and R" are the same or different and represent C₁₋₆ alkyl groups, preferably C₁₋₄ alkyl groups, as defined previously. As used herein a C₁₋₆ acetylamino group is a C₁₋₆ acetyl group attached to an amino group as defined above. Similarly, a di(C₁₋₆)acetylamino group is an acetyl group bearing two C₁₋₆ alkyl groups and attached to an amino group as defined above.

As used herein, an alkoxy group is typically a said alkyl group attached to an oxygen atom. Similarly, an alkylthio group is typically a said alkyl group attached to a thio group.

In accordance with the present invention, an iron containing colloidal particle is conjugated to a targeting moiety.

The iron containing colloidal particle according to the present invention may be any suitable iron containing particle. Suitable particles include those comprising iron hydroxide, iron oxide hydrate, iron (II) oxide, iron (III) oxide, mixed iron oxide, metallic iron or mixtures thereof. In mixed iron oxides other metal oxides such as oxides of cobalt, nickel, manganese, beryllium, magnesium, calcium, barium, strontium, copper, zinc, platinum, aluminium, chromium, bismuth, rare earth metals and mixtures thereof can be present. In preferred embodiments, the iron containing particle is an iron (II) or iron (III) oxide or an iron hydroxide or a mixture thereof. In a particularly preferred embodiment, the particle is iron oxide, in particular iron (III) oxide. The particles are preferably less than 1mm in size, preferably less than 100nm in size, and are typically less than 80nm, preferably less than about 50nm and may be as small as 5nm.

The particles preferably are cross-linked iron oxide particles (CLIOs). Such particles are described for example in Wunderbaldinger et al, Acad Radiol 2002 9 (supple 2) S304-S306. These particles comprise a core of iron oxide, that is preferably 3 to 10 nm, preferably 3 to 5 nm in size, and a dextran coat. Preferably the dextran coat is formed by crosslinking the dextran to form a dextran chain around the iron oxide core. Typically such particles may be produced by reacted dextran coated iron oxide particles in presence of epichlorohydrin and ammonia.

Such particles can be derivatised, for example with amine containing groups for conjugation to the targeting moieties according to the present invention. For example, amino groups of a dextran-coated iron oxide particle can react with a 2-cyanomethyl-containing compound to produce functionalised iron oxide particles.

The targeting moieties of the present invention are selected to target the conjugates to a selected cell type or tissue. In preferred embodiments, the targeting moieties are sugars, such as monosaccharides and oligosaccharides, and are selected to bind a cell surface receptor such as the selectin and lectin receptors. In a particularly preferred embodiment, the targeting moiety specifically targets a selectin or lectin, preferably a selectin, and most preferably selectin E or selectin P or both. Preferably the targeting moieties are selected to bind to animal lectins, as opposed to plant lectins.

The targeting moiety is preferably a sugar. The targeting moiety is specifically chosen in order to interact with a site of interest. This allows specific targeting of a contrast agent to a site or an area of interest. The targeting moiety is thus named because it is capable of targeting and interacting with a site of interest. The targeting moiety must therefore interact with a site, e.g. a receptor, in such a way as to direct the conjugate to that particular receptor. The targeting moiety may be a monosaccharide, but is more preferably an oligosaccharide. The nature of the targeting moiety can be strictly controlled such that the targeting moieties have a defined structure. In contrast, in the prior art moieties which are capable of targeting are often undefined long chain polymers which do not have such a precise structure..

Targeting monosaccharide moieties can include fused bicyclic units. When oligosaccharides are employed, these comprise from 2 to 15 saccharide units, more preferably from 2 to 10 saccharide units, for example from 2 to 6 saccharide units. Preferred oligosaccharides include those having 2 saccharide units (disaccharides), 3 saccharide units (trisaccharides) or 4 saccharide units (tetrasaccharides). The saccharide units used in the oligosaccharides are chosen depending upon the target to which the conjugate is aimed. However, suitable building blocks which make up the oligosaccharides include hexoses such as glucose, galactose and mannose, deoxyhexoses such as fucose and rhamnose, and pentoses such as arabinose and xylose.

The saccharides used in the invention can be functionalised. For examples, one or more hydroxy groups on an unfunctionalised saccharide may be replaced by a group selected from hydrogen, halogen, mercapto, C₁₋₆ alkoxy, C₁₋₆ alkylthio, -COOR' where R' is hydrogen or a C₁₋₆ alkyl group, amino, C₁₋₆ alkylamino, di(C₁₋₆)alkylamino, C₁₋₆ acetylamino, di(C₁₋₆)acetylamino and phosphate. Suitable phosphate groups include those of formula -O-PO(OH)₂. When the substituent is halogen, it is preferably fluorine. When the substituent is an alkoxy group it is preferably methoxy or ethoxy. When the substituent is -COOR', preferably R' is hydrogen, methyl or ethyl. When the substituent is a C₁₋₆ acetylamino group it is preferably a group of formula -NHCOR'
wherein R' is a C₁₋₆ alkyl group, preferably methyl or ethyl.

Thus, suitable saccharide units which can be present either alone or in oligosaccharides include monosaccharides such as GalNAc (N-acetyl galactosamine), GalUA (galacturonic acid), GlcNAc (N-acetyl glucosamine), GlcUA (glucuronic acid), IdUA (iduronic acid) and sialic acids such as NANA (neuraminic acid).

Particularly preferred saccharide units include N-acetyl glucosamine, fucose, galactose and sialic acid (Sia). When the targeting moiety is an oligosaccharide, the monosaccharides which comprise the oligosaccharide are the same or different, and preferably at least two of these monosaccarides are different. For example, targeting moieties may be Sialyl Lewis X (GIcNAc(-Fuc)-Gal-Sia), Lewis X (GlcNAc(-Fuc)-Gal) and GlcNAc-Gal.

For the avoidance of doubt, where an iron containing colloidal particle according to the invention is conjugated to more than one sugar targeting moiety, the sugar targeting moieties may be the same or different.

The targeting moieties may be selected to target a selected receptor depending on the diagnosis or imaging that is required. For example, the targeting to selectin using Sialyl Lewis X allows visualisation of areas of inflammation. The selectin receptor to which Sialyl Lewis X binds is up-regulated on the epithelium during inflammation. Thus targeting to this selectin can show areas of inflammation. Targeting to other lectins can be useful for example where such lectins are up-regulated in tumours.

The conjugates of the invention may be in the form of pharmaceutically acceptable salts, with the salts being formed with groups on the targeting moiety of the conjugate. A pharmaceutically acceptable salt is a salt with a pharmaceutically acceptable acid or base. Pharmaceutically acceptable acids include both inorganic acids such as hydrochloric, sulphuric, phosphoric, diphosphoric, hydrobromic or nitric acid and organic acids such as citric, fumaric, maleic, malic, ascorbic, succinic, tartaric, benzoic, acetic, methanesulphonic, ethanesulphonic, benzenesulphonic or p-toluenesulphonic acid. Pharmaceutical acceptable bases include alkali metal (e.g. sodium or potassium) and alkaline earth metal (e.g. calcium or magnesium) hydroxides and organic bases such as alkyl amines, aralkyl amines or heterocyclic amines.

Tautomers of the conjugates of the invention defined above also form part of the invention. Also, conjugates defined above containing one or more chiral centre may be used in enantiomerically or diasteroisomerically pure form, or in the form of a mixture of isomers. The presence of one asymmetric carbon atom in conjugates of the invention will give rise to enantiomers. The presence of more than one asymmetric carbon atom will give rise to diastereoisomers, each of which consists of two enantiomers, with the appropriate (*R*)- or (*S*)- stereochemistry at each chiral centre. For the avoidance of doubt, the chemical structures depicted herein are intended to embrace all stereoisomers of the conjugates shown, including racemic and non-racemic mixtures and pure enantiomers and/or diastereoisomers.

For the avoidance of doubt, the conjugates of the invention can, if desired, be used in the form of solvates.

The conjugates of the invention can be formulated for use by combining in a formulation with a pharmaceutically acceptable excipient. The formulations are typically prepared following conventional methods and are administered in a pharmaceutically suitable form.

The dosages in which the formulations according to the invention are administered will vary according to the mode of use and the route of use, as well as to the requirements of the patient.

Solid oral forms of the formulations of the invention may contain, together with the conjugated particles themselves, diluents, e.g. lactose, dextrose, saccharose, cellulose, corn starch or potato starch; lubricants, e.g. silica, talc, stearic acid, magnesium or calcium stearate, and/or-polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non toxic and pharmacologically inactive substances used in pharmaceutical formulations. Such formulations may be manufactured in known manner, for example, by means of mixing, granulating, tableting, sugar coating, or film coating processes.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspension or solutions for intramuscular injections may contain, together with the conjugated particles of the invention, a pharmaceutically acceptable carrier, e.g. sterile water, olive oil, ethyl oleate, glycols, e.g. propylene glycol, and if desired, a suitable amount of lidocaine hydrochloride.

Solutions for injection or infusion may contain as carrier, for example, sterile water or preferably they may be in the form of sterile, aqueous, isotonic saline solutions. Most preferably the formulation comprises a conjugate according to the invention and saline.

The conjugates according to the present invention can be used as contrast agents in methods of imaging. The agents of the present invention are particularly useful as contrast agents using magnetic resonance imaging (MRI). The agents can be delivered to the patient under investigation by any suitable route, but are typically provided by injection, usually intravenous injection. The agents of the present invention may cross the blood brain barrier and so may be particularly useful in the monitoring or diagnosis of conditions affecting the brain. In a preferred embodiment, the contrast agents are used in the monitoring and diagnosis of inflammation. The agents may also be useful, depending on the targeting moiety selected for the monitoring and diagnosis of tumours. Such agents will preferably target tumours.

In a particularly preferred embodiment according to the present invention, the agents are used in the monitoring and diagnosis of inflammation in the brain, and are particularly useful in the diagnosis of multiple sclerosis. The agents of the present invention have the advantage of crossing the blood brain barrier and so may be used to provide an indication of inflammation and other disorders in the brain before the condition is advanced.

### Processes:

Processes to prepare conjugates according to the present invention require a protected thio-cyanomethyl intermediate to be converted to a 2-methyl-2-imido-linker (IME) system in order for subsequent conjugation to take place.

It is possible to react a protected monosaccharides in an excess of sodium methoxide and methanol in order to deprotect the monosaccharide and simultaneously generate the IME system, hence activating the linker. This is shown along the top line of Scheme 1 below.

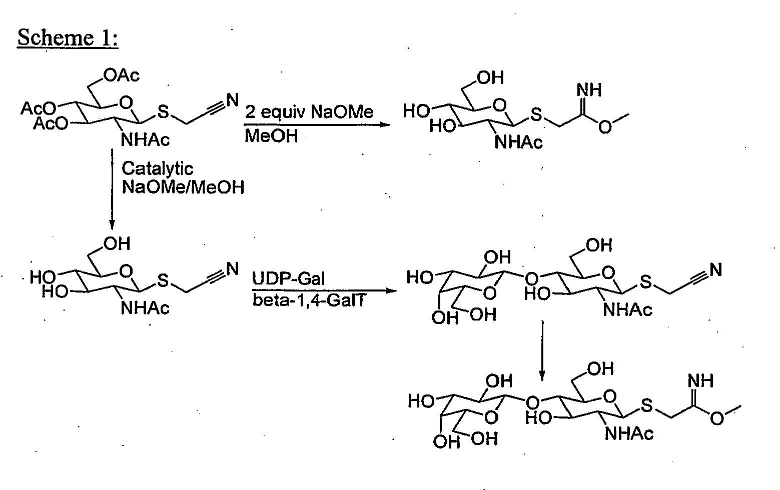

However, the product of this reaction is short lived, and it is furthermore currently difficult to add further targeting moieties onto this compound either before or after conjugation to an iron containing colloidal particle.

It has surprisingly been found that the deprotection and activation steps of the above reaction can be uncoupled. Thus, the thio-cyanomethyl intermediate can be deprotected first, then subsequently extended by the addition of further targeting moieties without the IME system being generated. The selective deprotection can be carried out using any reaction known to those skilled in the art. For example, selective deprotection of AcO groups can be carried out by a deacetylation reaction resulting in hydroxyl groups. For example, in Scheme 1 above, by using a catalytic amount of sodium methoxide and methanol, the AcO groups on the starting material can be deprotected by conversion to hydroxy groups, without the IME system being generated. The deprotected but unactivated compound can then be extended by the addition of a further targeting moiety and finally the intermediate can be activated by the conversion of the thio-cyanomethyl group to the IME system. This IME-terminated compound can then be conjugated to an iron containing colloidal particle. Alternatively, the cyanomethyl-containing compounds can be reacted with amine groups on the dextran coat of an iron oxide particle in order to produce a functionalised iron oxide particle.

The number of further carbohydrate-based moieties added to the starting material can vary. However, it is preferred that between one and four further moieties are added, more preferably one or two, and most preferably just one. These further carbohydrate-based moieties are preferably saccharides, most preferably monosaccharides. Furthermore, the number of targeting groups present in the starting material can vary, although preferably the starting material contains one or two, more preferably one, saccharide group. Accordingly, it is preferred that a monosaccharide is reacted as shown above in Scheme 1 by the addition of a further monosaccharide in order to produce an IME-terminated disaccharide which can subsequently be used to prepare the conjugates according to the invention.

Accordingly, the invention provides a process for preparing an intermediate which comprises:
(a) providing a compound of formula (I):

   R¹-S-CH₂CN (I)

   wherein R¹ is an acetylated carbohydrate-based moiety;
(b) selectively deprotecting the compound of formula (I) in order to convert the acetylated groups to hydroxy groups;
(c) reacting the product of step (b) with a carbohydrate processing enzyme in order to extend the compound of formula (I) by the addition of one or more carbohydrate-based moieties R²; and
(d) activating the product of step (c) in order to produce a compound of formula (II):

   (R²)ₙ-R¹-S-CH₂-C(NH)-O-Me (II)

   wherein n is the number of carbohydrate-based moieties R² added in step (c) and is an integer of from 1 to 10.

Preferably R¹ group is a saccharide as defined earlier wherein the hydroxy groups -OH have been protected as acetyloxy groups CH₃COO-. The R¹ group can be a monosaccharide or an oligosaccharide such as a disaccharide. When R¹ is a monosaccharide preferred carbohydrate-based moieties include glucosamines. When R¹ is an oligosaccharide, preferred carbohydrate-based moieties which are present include galactopyranosides and glucopyranosides. Most preferably R¹ is a monosaccharide.

The carbohydrate processing enzyme is any suitable enzyme which can be used to extend the carbohydrate-based moiety R¹. The carbohydrate processing enzyme extends the carbohydrate-based moiety by the addition of one or more further carbohydrate-based moieties R². For example, galactosyltransferase and UDP-galactone can be used, as well as sialyltransferase such as α-2,3-sialyltransferase and CMP-sialic acid, as well as fucosyltransferase such as α-1,3-fucosyltransferase and GDP-fucose.

The integer n may range from 1 to 6. However, preferably n is from 1 to 4, more preferably 1 or 2, most preferably 1. Thus, it is preferred that a single further carbohydrate-based moiety is added. Preferably this further carbohydrate-based moiety is a monosaccharide. Thus, in a preferred embodiment the invention provides a disaccharide intermediate of formula (III):

R²-R¹-S-CH₂-C(NH)-O-Me (III)

wherein R¹ and R² are each monosaccharide moieties. The monosaccharides are preferably as defined earlier.

The intermediates of formulae (II) and (III) described above can be further reacted with an iron containing colloidal particle, such as a cross-linked iron oxide particle, in order to prepare an imaging agent according to the present invention. Preferably the cross-linked iron oxide (CLIO) particle is functionalised in order to promote this reaction. Any suitable functional group can be present provided it is capable of reacting with the IME linker group in order to form a bond between the intermediate and the CLIO particle. The functional group is preferably present on the dextran coating of a cross-linked iron oxide particle. Preferred functional groups are amines, in particular -NH₂.

Thus, the invention further provides a process for preparing a conjugate according to the invention which comprises reacting an intermediate of formula (II) or formula (III) as defined above with an iron containing colloidal particle. Preferred R¹ and R² groups and preferred iron containing colloidal particles are described earlier. Particularly preferred iron containing colloidal particles are cross-linked iron oxides bearing amino functional groups such as -NH₂.

Once a conjugate has been prepared as described above, it may then be further extended by the action of further carbohydrate processing enzymes. Thus, the process described in the paragraph above can further comprise the step of reacting the conjugate with a carbohydrate processing enzyme in order to add one or more further carbohydrate-based moieties. The carbohydrate processing enzymes include those mentioned above. For example, in order to extend a disaccharide-functionalised CLIO particle suitable reactants include CMP-Sialic acid, GDP-Fucose, α-2,3-sialyltransferas and α-1,3-fucosyltransferase can be used in order to add further carbohydrate-based moieties.

A further aspect of the invention relates to the preparation of the thio-cyanomethyl starting materials. The invention therefore provides a method for preparing a compound of formula (I) as defined above, comprising:
(a) providing a compound of formula (IV):

   R¹-O-C₆H₄-OR³ (IV)

   wherein R³ is a C₁₋₆ alkyl group;
(b) reacting the compound of formula (IV) with ammonium cerium nitrate to produce a compound of formula (V):

   R¹-OH (V)
(c) reacting the compound of formula (V) with a thionyl halide, preferably thionyl chloride, and subsequently reacting with thiourea to produce a compound of formula (VI):

   R¹-S-C(=NH)-NH₂ (VI)
(d) reacting the compound of formula (VI) with chloroacetonitrile under conditions sufficient to produce a compound of formula (I).

Preferably R³ is C₁₋₄ alkyl, more preferably methyl or ethyl, most preferably methyl. Preferably R¹ is an oligosaccharide, more preferably a disaccharide, trisaccharide or tetrasaccharide, more preferably a disaccharide or trisaccharide. When R¹ is an oligosaccharide, the step of reacting a compound of formula (V) with a thionyl halide and subsequently with thiourea to produce a compound of formula (VI) has not previously been performed. Furthermore, it is preferred that the compound of formula (IV) comprises a protected disaccharide with NHAc on position 2 and a *p-*methoxyphenyl (PMP) group on position 1.
The invention will be described in the Examples which follow.

### EXAMPLES

### Example 1: Synthesis of 1-chloro-2,3,4,6-tetraacetyl glucosamine (1)

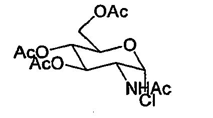

To a solution of N-Acetyl-D-glucosamine (4.97, 0.022 mol), acetyl chloride was added (10ml, 0.14 mol) and stirred under Argon for 18h, after which the reaction mixture had turned into a pink gel. 20 ml of dichloromethane was added and the reaction mixture was poured into 20 ml of ice water. The organic layer was washed three times with double volumes of bicarbonate of soda, dried over MgSO₄ and concentrated in vacuo. The resulting brown oil further purified by flash chromatography (ethyl acetate) to yield 4.05g (0.011 mol; 50% yield) of a white solid.

NMR Data: δ_{H} (400MHz, CDCl₃) 2.00 (3H, s, CH₃), 2.07 (6H, s 2 CH₃), 2.12 (3H, s, CH₃), 4.12-4.23 (3H, m, H-6, H-6', H-5), 4.52-4.57 (1H, m, H-2), 5.23 (1H, dd, J 9.7 Hz, H-4), 5.31-5.36 (1H, dd, J 9.42 Hz, H-3), 5.79 (1H, d, J 8.7 Hz, NH), 6.20 (1H, d, J 3.7 Hz, H-1)

### Example 2: Syntheses of 1-thiourea-2,3,4,6-Tetraacelyl-2-glucosamine (2)

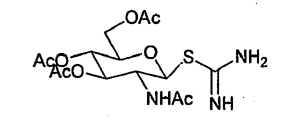

1-chloro-2,3,4,6-tetraacetyl glucosamine (1) (4.99g, 0.014 mole) was dissolved in 50ml of dry acetone. Thiourea (2.01g, 0.026 mole) was added and the reaction was heated to reflux temperature for 3 hours, after which a white solid had precipitated out of the reaction mixture. The white solid was filtered, the residue was washed with ethanol (400ml) and dried in vacuo to yield a white solid (5.31g, 85% yield). M/S: [ESI]+: 406, [ESI]-: 440 [M+Cl]-

### Example 3: Synthesis of 1-thio-S-cyanomethyl-2,3,4,6-tetraacetyl-2-glucosamine (3)

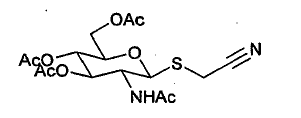

1-thiourea-2,3,4,6-Tetraacetyl-2-glucosamine (2) (20.01g, 0.045mol) was dissolved in 400ml of a 1:1 water:acetone mixture. Sodium bisulfite (9.36g, 0.09 mole) and potassium carbonate (6.22g, 0.045 mole) were added. 25 ml of chloroacetonitrile (0.47 mole) was also added and the reaction mixture was stirred for 3 hours prior to 500ml of ice water being added. The reaction was then reacted for another 2 hours. The reaction mixture was extracted with 11 of dichloromethane and the organic layer was washed with three half volumes of a saturated sodium chloride solution, over MgSO₄ and concentrated in vacuo. The resulting crude was recrystallised from hot ethanol to yield 10.51g (0.026 mole, 58%) of a white solid.

### Example 4: Synthesis of 1-thio-S-cyanomethyl-N-acetyl-D-glucosamine (4)

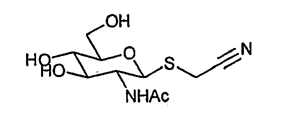

1-thio-S-cyanomethyl-2,3,4,6-tetraacetyl-2-glucosamine (3) (10.41g, 0.026 mole) was dissolved in 400ml of dry methanol. 1 wt% of sodium methoxide (100mg, 0.0019 mole) was added. The reaction mixture was reacted for 18h under argon, after which time TLC (ethyl acetate) indicated the complete consumption of starting material (R_{f} 0.5) and the formation of a single product (R_{f} 0.1). The reaction mixture was concentrated in vacuo. And the white solid formed was recrystallised from hot methanol to yield 3.5g (49% yield) of a white solid.
mp: 173.5-174.2 °C. [α]²⁵_{D}: -75.7 (c = 1.0, H₂O). IR (KBr disc) 3480.5cm⁻¹ OH); 3295.0 cm⁻¹ (NH); 2979.6cm⁻¹ (C-H); 2257.1cm⁻¹ (C≡N); 1651.5cm⁻¹ (C=O). δ_{H} (400MHz, DMSO): 1.81 (3H, s, CH₃), 3.07-3.18 (2H, m, H-4, H-5), 3.26-3.32 (1H, m, H-3), 3.42-3.48 (1H, m, H-6), 3.52-3.60 (1H, ddd, H-2, J_{2-NH} 9.07Hz, J₁₋₂ 10.4Hz, J₂₋₃ 10.04Hz), 3.67-3.72 (1H, m, H'-6), 3.71-3.86 (2H, 2 x d, CH₂, J 16.94), 4.48-4.50 (1H, d, H-1), 4.49-4.52 (1H, d, OH-6), 5.08-5.11 (2H, 2 x d, OH-3, OH-4), 7.87-7.89 (1H, d, NH). δ_{C} (100.2MHz, DMSO) 14.65 (CH₂), 23.76 (CH3), 62.14 (C-6), 71.30, 82.28 (C-4, C-5), 75.83 (C-3), 83.97 (C-1), 118.95 (CN), 170.18 (COMe). HRMS (ES⁻ )C₁₀H₁₅N₂O₅S requires 275.0702, found 275.0698.

### Example 5: Synthesis of 4-O-[2,3,4,6-tetra-O-acetyl-β-d-galactopyranosyl]-2-deox-1-para-methoxyphenyl-2-acetimido-3,6-di-O-acetyl-β-d-glucopyranoside (5)

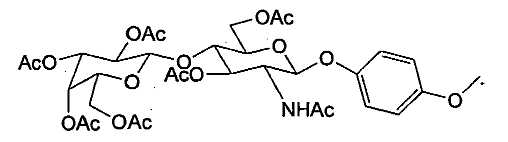

4-O-[2,3,4,6-tetra-O-acetyl-β-d-galactopyranosyl]-2-deoxy-1-paramethoxyphenyl-2-phthalimido-3,6-di-O-benzyl-β-d-glucopyranoside (1.01 g, 1.2 mmol) was dissolved in 100 mL tetrahydrofuran. Pearlman's catalyst was added and the suspension was placed under an atmosphere of hydrogen. After 2.5 hours TLC (15% 40-60°C petroleum ether in ethyl acetate) indicated the complete consumption of starting material (R_{f} 0.9) and the formation of a single product (R_{f} 0.8). The mixture was filtered through celite and concentrated in vacuo to yield 958 mg of a clear foam. This foam was dissolved in methanol (150 mL) and ethylene diamine (50 mL) was added. The mixture was heated to reflux for 17 hours and concentrated in vacuo, the crude concentrate was dissolved in pyridine (110 mL) and after the addition of acetic anhydride (100 mL) was reacted for 14 hours after which the reaction was concentrated in vacuo prior to subsequent redissolution in dichloromethane (100 mL). The organic layer was washed with a saturated solution of bicarbonate of soda (100 mL) and a saturated sodium chloride solution (50 mL) prior to drying over anhydrous magnesium sulfate and concentration in vacuo. The crude product was purified by flash silica chromatography (ethyl acetate) to yield 750 mg of a white foam (1.01 mmol, 84% yield).
¹H NMR (500 MHz, CDCl₃) δ = 1.98, 2.00, 2.06, 2.07, 2.07, 2.11, 2.15 (7 x s, 7 x 3H, 7 x C(O)CH₃), 3.76 (s, 3H, OCH₃), 3.76-3.80 (m, 1H, Hₐ-5), 3.87 (app t, J 7.7 Hz, Hₐ-4), 3.91 (app dt, J₄₋₅ 0.9 Hz, J 7.1 Hz, H_{b}-5), 4.11 (dd, 1H, J₅₋₆ 7.1 Hz, J6-6' 13.4 Hz, H_{b}-6), 4.14 (dd, J₅₋₆, 7.1 Hz, H_{b}-6'), 4.18 (dd, 1H, J₅₋₆ 6.0 Hz, J_{6-6'} 12.0 Hz, Hₐ-6), 4.30 (app dt, 1H, J₁,₂ 7.0 Hz, J 9.0 Hz, Hₐ-2), 4.50 (dd, 1H, J_{5-6'} 3.4 Hz, Hₐ-6'), 4.52 (d, 1H, J1-2 7.9 Hz, H_{b}-1), 4.97 (d, 1H, Hₐ-1), 5.00 (dd, 1H, J₂₋₃ 10.6 Hz, J₃₋₄ 3.4 Hz, H_{b}-3), 5.14 (dd, 1H, H_{b}-2), 5.16 (dd, 1H, J₂₋₃ 8.8 Hz, J₃₋₄ 7.7 Hz, Hₐ-3), 5.37 (dd, 1H, H_{b}-4), 5.91 (d, 1H, J_{NH-H-2} 9.4 Hz, N-H), 6.79-6.95 (m, 4H, Ar-H). ¹³C NMR (125.6 MHz, CDCl₃) δ = 20.1, 20.5, 20.5, 20.6, 20.7, 23.1 (C(O)CH₃), 52.5 (Cₐ-2), 55.5 (OCH3), 60.7 (C_{b}-6), 62.4 (Cₐ-6), 66.7 (C_{b}-4), 69.0 (C_{b}-2), 70.6, 70.7 (C_{b}-3, C_{b}-5), 71.6 (Cₐ-3), 72.6 (Cₐ-5), 75.1 (Cₐ-4), 99.8 (Cₐ-1), 100.8 (C_{b}-1), 114.4, 118.1 (CH-Ar), 150.9, 155.3 (C-Ar), 169.4, 169.9, 170.0, 170.1, 170.2, 170.2, 170.3 (C(O)O). HRMS (ES⁺) C₃₃H₄₄NO₁₈ requires 742.2558, found 742.2552

### Example 6: Synthesis of 4-O-[2,3,4,6-tetra-O-acetyl-β-d-galactopyranosyl]-2-deox-1-para-methoxyphenyl-2-acetimido-3,6-di-O-acetyl-α-d-glucopyranose (6)

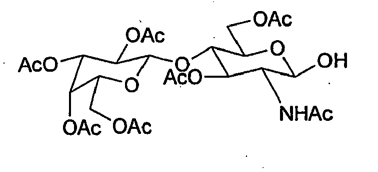

Compound (5) (696 mg, 0.96 mmol) was dissolved in 9:1 acetonitrile/water (15 mL). Ammonium cerium nitrate (2.6 g, 4.7 mmol, 5 equivalents) was added and the mixture was reacted at room temperature for 1 hour after which TLC (ethyl acetate) indicated the disappearance of starting material (R_{f} 0.3) and the formation of a single product (R_{f} 0.05). Water (100 mL) was added to the reaction mixture and the aqueous layer was extracted 3 times with dichloromethane. The combined organic layers were dried over anhydrous magnesium sulfate and concentrated in vacuo. The crude product was purified by flash silica chromatography (ethyl acetate, 41:1 ethyl acetate:methanol) to yield 244 mg of a brown oil (0.38 mmol, 40% yield).

¹H NMR (400 MHz, CDCl₃) δ = 1.97, 2.01, 2.06, 2.07, 2.08, 2.12, 2.15 (7 x s, 7 x 3H, 7 x C(O)CH₃), 3.77 (app t, 1H, J 9.4 Hz, Hₐ-4), 3.88 (app dt 1H, J₄₋₅ 1.1 Hz, J 6.5 Hz, H_{b}-5), 4.02, 4.17 (m, 4H, Hₐ-5, Hₐ-6, H_{b}-6, H_{b}-6'), 4.26 (app dt, 1H, J₁₋₂ 3.5 Hz, J 10 Hz, Hₐ-2), 4.44 (d, 1H, J₁₋₂ 7.7 Hz, H_{b}-1), 4.46 (m, 1H, Hₐ-6'), 5.01 (dd, 1H, J₃₋₄ 3.4 Hz, J₂₋₃10.5 Hz, H_{b}-3), 5.10 (dd, 1H, J₂₋₃10.5 Hz, H_{b}-2), 5.20 (d, 1H, Hₐ-1), 5.37 (dd, 1H, H_{b}-4), 5.52 (dd, 1H, J₃₋₄ 9.2 Hz, J₂₋₃10.8 Hz, Hₐ-3), 6.61 (d, 1H, J_{NH-2} 9.7 Hz, NH) ¹³C NMR (partial) (100.6 MHz, CDCl₃) δ = 20.49, 20.63, 20.74, 20.86, 20.94, 21.04, 22.92 (C(O)CH₃), 51.99 (Cₐ-2), 60.62 (C_{b}-6), 62.07 (Cₐ-6), 66.55 (C_{b}-4), 68.27 (Cₐ-5), 69.58 (C_{b}-2), 70.40 (C_{b}-3), 70.64 (C_{b}-5), 71.05 (Cₐ-3), 75.83 (Cₐ-4), 91.54 (Cₐ-1), 100.94 (C_{b}-1). HRMS (ES⁺) C₂₆H₃₈NO₁₇ requires 636.2140, found 636.2143.

### Example 7: Synthesis of 4-O-[2,3,4,6-tetra-O-acetyl-β-d-galactopyranosyl]-2-deoxy-1-thio-S-cyanomethyl-2-acetimido-3,6-di-O-acetyl-β-d-glucopyranose (7)

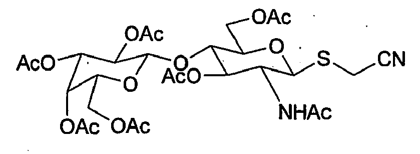

Compound (6) (211 mg, 0.33 mmol) was dissolved in anhydrous dichloromethane (5 mL) and anhydrous toluene (5 mL). Thionyl chloride (5 mL) was added and the mixture was stirred under an argon atmosphere at room temperature for 12 hours after which the mixture was concentrated in vacuo and purified by flash silica chromatography (4:1 ethyl acetate:40-60°C petroleum ether) to yield 110 mg of a brown oil.

The oil was dissolved in acetone (10 mL). Thiourea (436 mg, 5.7 mmol, 17 equivalents) was added and the mixture was heated to reflux in an open vessel microwave for 20 minutes prior to being concentrated in vacuo. The residue was redissolved in dichloromethane and filtered. The remaining residue was washed with an additional portion of dichloromethane and the combined organic layers were concentrated and purified by flash silica chromatography (200 mL ethyl acetate, followed by 500 mL of 4:1 dichloromethane:methanol; the second charge was collected) to yield 50 mg of a white foam.

This foam was dissolved in acetone (5 mL) and water (5 mL). Sodium metabisulfite (54.6 mg) and potassium carbonate (17.6 mg) were added, as was chloroacetonitrile (10 µL). The mixture was stirred for 2.5 hours when TLC (ethyl acetate) indicated the complete consumption of starting material (R_{f} 0.1) and the formation of a single product (R_{f} 0.5). Dichloromethane was added and the combined organic layer was washed with a saturated solution of bicarbonate of soda (50 mL), a saturated sodium chloride solution (20 mL) prior to being dried over anhydrous magnesium sulfate and concentrated in vacuo. Further purification by flash silica chromatography (ethyl acetate) to yield 17.7 mg of a pale oil (0.03 mmol, 8% yield) ¹H NMR (400 MHz, CDCl₃) δ =1.98, 1.98, 2.07, 2.07, 2.10, 2.14, 2.16 (7 x s, 7 x 3H, 7 x x C(O)CH₃), 3.27 (d, 1H 2J 17.0 Hz, CHCN), 3.64-3.68 (m, 1H, Hₐ-5), 3.68 (d, 1H, CH'CN), 3.84 (app t, 1H, J 9.2 Hz, Hₐ-4), 3.90 (app dt, 1H, J₄₋₅ 0.8 Hz, J 7.0 Hz, H_{b}-5), 4.07-4.14 (m, 3H, Ha-6, H_{b}-6, H_{b}-6'), 4.21 (app q, 1H, J 10.0 Hz, Hₐ-2), 4.52 (d, 1H, J₁₋₂ 7.9 Hz, H_{b}-1), 4.56 (dd, 1H, J₅₋₆ 2.0 Hz, J_{6-6'} 12.0 Hz, Hₐ-6), 4.65 (d, 1H, J₁₋₂ 10.3 Hz, Hₐ-1), 4.98 (dd, 1H, J₃₋₄ 3.4 Hz, J₂₋₃ 10.5 Hz, H_{b}-3), 5.09 (dd, 1H, J 10.2 Hz, J 9.0 Hz, Hₐ-3), 5.11 (dd, 1H, J₂₋₃10.6 Hz, H_{b}-2), 5.37 (dd, 1H, J₄₋₅ 0.8 Hz, H_{b}-4), 5.81 (d, 1H, J_{NH-2} 9,4 Hz, NH). HRMS (ES⁺) C₂₈H₃₉N₂O₁₆S requires 691.202, found 691.2015.

### Example 8: Synthesis of 4-O-[2,3,4,6-tetra-O-acetyl-β-d-galactopyranosyl]-3-(2,3,4-triO-acetyl-α-d-fucosyl)-2-deoxy-1-(4-methoxyphenyl)-2-acetimido-6-O-acetyl-β-d-glucopyranoside (8)

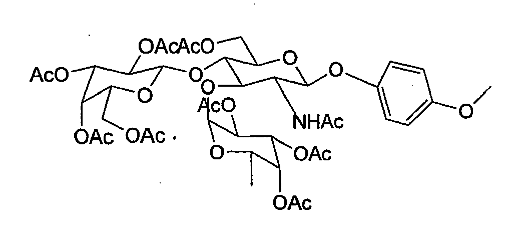

4-O-[2,3,4,6-tetra-O-acetyl-β-d-galactopyranosyl]-2-deoxy-1-paramethoxyphenyl-2-phthalimido-3,6-di-O-benzyl-β-d-glucopyranoside (1.7 g, 1.35 mmol) was dissolved in tetrahydrofuran. Pearlman's catalyst was added and the mixture was evacuated prior being placed under a hydrogen atmosphere. The mixture was stirred for 19 hours after which TLC (ethyl acetate) indicated the complete consumption of starting material (R_{f} 0.9) and the formation of a single product (R_{f} 0.3). The reaction mixture was concentrated in vacuo and co-evaporated with ethanol twice. The resulting oil was re-dissolved in methanol (mL) prior to the addition of 2 mL of ethylene diamine. The mixture was heated to 60°C for 22 hours prior to being concentrated in vacuo. The resulting yellow oil was then redissolved in acetic anhydride (4 mL) and pyridine (2 mL) and reacted for 18 hours under an argon atmosphere. The crude mixture was concentrated in vacuo followed by co-evaporation with toluene twice and ethanol once. The resulting oil was purified by flash column chromatography (ethyl acetate) to yield 5 as a white amorphous solid (814 mg, 64% yield)
¹HNMR (500 MHz, CDCl₃) δ = 1.21 (d, 3H, J₅₋₆ 6.5 Hz, H_{c}-6), 1.95-2.20 (m, 27H, 9 x C(O)CH₃), 3.74-3.77 (m, 1H, Hₐ-5), 3.75 (s, 3H, OCH₃), 3.87-3.92 (m, 2H, Hₐ-4, H_{b}-5), 4.05-4.16 (m, 2H, Hₐ-2, Hₐ-3), 4.23 (dd, 1H, J₅₋₆ 5.9 Hz, J_{6-6'} 11.9 Hz, Ha-6), 4.28 (dd, 1H, J₅₋₆ 7.5 Hz, J_{6-6'} 11.4 Hz, H_{b}-6), 4.44 (dd, 1H, J₅₋₆, 6.4 Hz, H_{b}-6), 4.48 (d, 1H, J₁₋₂ 8.1 Hz, H_{b}-1), 4.59 (dd, 1H, J_{5-6'} 3.7 Hz, Hₐ-6'), 4.69-4.73 (app. q, 1H, H_{c}-5), 5.02 (dd, 1H, J₂₋₃ 10.5 Hz, J₃₋₄ 3.5 Hz, H_{b}-3), 5.07 (dd, 1H, J₁₋₂ 3.9 Hz, J₂₋₃ 11.0 Hz, H_{c}-2), 5.08 (d, 1H, J₁₋₂ 8.7 Hz, Hₐ-1), 5.12 (dd, 1H, H_{b}-2), 5.24 (dd, 1H, J₃₋₄ 3.4 Hz, H_{c}-3), 5.38 (app. d, 1H, H_{c}-4), 5.43 (dd, 1H, J₄₋₅ 0.39 Hz, H_{b}-4), 5.47 (d, 1H, H_{c}-1), 5.90 (d, 1H, J_{NH-2} 8.8 Hz, NH), 6.77-6.93 (m, 4H, Ar-H). ¹³C NMR (125.6 MHz, CDCl₃) δ = 15.7 (C_{c}-6), 20.4, 20.5, 20.5, 20.5, 20.6, 20.6, 20.8, 23.2 (OC(O)CH3), 55.5 (OCH3), 60.7 (C_{b}-6), 62.4 (Cₐ-6), 64.5 (C_{c}-5), 66.6 (C_{b}-4), 67.9, 68.0 (C_{c}-5, C_{c}-2), 68.7 (C_{b}-2), 70.5 (C_{b}-3), 71.1, 71.1, 74.0 (Cₐ-4, C_{b}-5, C_{c}-4), 72.4, 72.7 (Cₐ-2, Cₐ-3, Cₐ-5), 94.9 (C_{c}-1), 99.4 (Cₐ-1), 100.2 (C_{b}-1), 114.4, 118.2 (CH-Ar), 151.1, 155.3 (C-Ar), 169.4, 169.7, 169.8, 170.1, 170.2, 170.4, 170.5, 170.6, 171.0 (C(O)O)

### Example 9: Synthesis of 4-O-[2,3,4,6-tetra-O-acetyl-β-d-galactopyranosyl]-3-(2,3,4-triO-acetyl-α-d-fucosyl)-2-deoxy-2-acetimido-6-O-acetyl-β-d-glucopyranose (9)

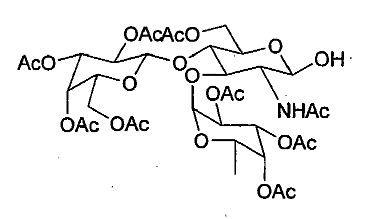

Compound (8) (695 mg, 0.72 mmol) was dissolved in 9:1 acetonitrile:water (50 mL). Ammonium cerium nitrate (2.4 g, 6 equivalents was added) and the mixture was stirred at room temperature for 1.5 h. The solvent was subsequently removed *in vacuo* and the residue was redissolved in chloroform (500 mL). The organic layer was washed with bicarbonate of soda (300 mL) and brine (200 mL), then dried over magnesium sulfate and filtered. The crude mixture was further purified by flash column chromatography to yield 295.9 mg of a pale oil, as well as 119.1 mg of a product believed to be the oxazoline equivalent of the product trisaccharide.

### Example 10: Synthesis of 4-O-[2,3,4,6-tetra-O-acetyl-β-d-galactopyranosyl]-3-(2,3,4-tri-O-acetyl-α-d-fucosyl)-1-thioureyl-2-deoxy-2-acetimido-6-O-acetyl-β-d-glucopyranoside (10)

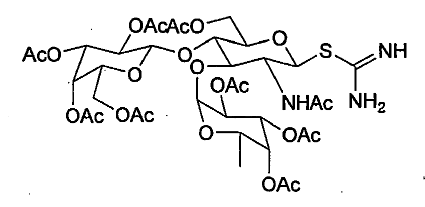

Compound (9) (280 mg, 0.32 mmol) was dissolved anhydrous dichloromethane (10 mL) and anhydrous toluene (10 mL). Thionyl chloride (2 mL) was added and the mixture was stirred at room temperature for 3 h prior to being concentrated *in vacuo.* The crude product was purified by flash silica chromatography (3:1 ethyl acetate/40-60°C petroleum ether) to yield 191 mg of a yellow oil.

To a solution of the compound in acetone was added thiourea (949 mg, 12 mmol) and the mixture was stirred under argon for 4 hours, prior to being concentrated *in vacuo.* The crude product mixture was redissolved in 250 mL of dichloromethane and filtered. The residue was washed with an additional 250 mL of dichloromethane. The combined organic washes were concentrated and dry-loaded onto silica. Impurities were first eluted by washing with 500 mL of ethyl acetate. The product was then eluted in 4:1 dichloromethane/methanol to give 128 mg of thiouroneum salt.

### Example 11: Synthesis of 4-O-[2,3,4,6-tetra-O-acetyl-β-d-galactopyranosyl]-3-(2,3,4-tri-O-acetyl-α-d-fucosyl)-1-thio-S-cyanomethyl-2-deoxy-2-acetimido-6-O-acetyl-β-d-glucopyranoside (11)

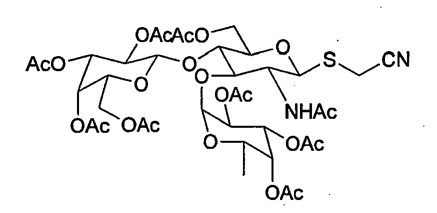

Compound (10) (107 mg, 0.18 mmol) was dissolved in acetone (10 mL) and water (10 mL). Added were sodium metabisulfite (49.5 mg, 2 equivalents), potassium carbonate (17.3 mg, 1 equivalent) and chloroacetonitrile (70 µL, 6 equivalents). The reaction mixture was stirred for 3 hours at room temperature. The mixture was extracted with dichloromethane (3 x 30 mL) and the combined organic layer was washed with a saturated sodium chloride solution (50 mL), dried over anhydrous magnesium sulfate, filtered and concentrated *in vacuo.* The crude product was further purified by flash silica chromatography (3:1 ethyl acetate/40-60°C petroleum ether) to yield 50.4 mg of a clear oil.

### Example 12: Synthesis of 1,3,4,6,-O-Acetyl-2-deoxy-2-phthalimido-β-D-glucopyranoside (12)

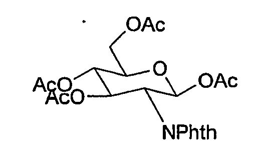

D-Glucosamine hydrochloride (35.2 g, 0.16 mol) was dissolved in anhydrous methanol (250 ml). Sodium methoxide (10.02 g, 0.19 mol) was added and the mixture was stirred under a nitrogen atmosphere for 10 minutes. The solid was removed by filtration and a portion of phthalic anhydride (15.2 g, 0.10 mol) was added to the filtrate. A white solid formed in the reaction mixture. A second portion of phthalic anhydride (15.8 g, 0.10 mol) was added as was triethylamine (26 mL) prior to the suspension being heated to 50 °C for 45 minutes. The reaction was then cooled in an ice bath for 1.5 hours. The resulting solid was filtered off and dried *in vacuo.*

The solid was redissolved in acetic anhydride (500 mL) and cooled again to 0°C. Pyridine (250 ml) was added and the reaction mixture was stirred at 0 °C for 40 minutes. After this time period the reaction was warmed to room temperature and stirred for a further 16 hours, after which the white suspension had turned to a clear red solution. To the reaction mixture 21 of ice water was added and the mixture was extracted with chloroform (2L). The organic layer was washed with a 1 M hydrogen chloride solution (1.5 L), a saturated bicarbonate of soda solution (1.5 L), water (1 L), a saturated solution of sodium chloride (1 L) prior to being dried over anhydrous magnesium sulfate and concentrated *in vacuo.* The product was further purified by flash silica chromatography (ethyl acetate) to yield 25.4 g (47% yield) of a white foam. [α]²⁵_{D} +70.7 (CHCl₃, c = 1.0) [lit. +68 (c = 0.5)]; ¹H NMR (400 MHz, CDCl₃) δ = 1.87, 2.01, 2.05, 2.12 (4 x s, 4 x 3H, C(O)CH₃), 4.03 (ddd, 1H, *J*₄₋₅ 10.2 Hz, *J*₅₋₆ 4.4 Hz, *J*_{5-6'} 2.12 Hz, H-5), 4.10-4.18 (m, 1H, H-6'), 4.38 (dd, 1H, *J*₅₋₆ 4.4 Hz, *J*₆₋₆.12.4 Hz), 4.48 (dd, 1H, *J*₁₋₂ 8.9 Hz, *J*_{2,3} 10.6 Hz, H-2), 5.22 (dd, 1H, *J*₃₋₄ 8.9 Hz, H-4), 5.90 (dd, 1H, H-3), 6.52 (d, 1H, H-1), 7.74-7.88 (m, 4H, 4 x Ar-H). *m*/*z* (ES⁻): 500 (100%, M + Na⁺).

### Example 13: Synthesis of 3,4,6-O-acetyl-2-deoxy-1-para-methoxyphenyl-2-phthalimido-β-D-glucopyranoside (13)

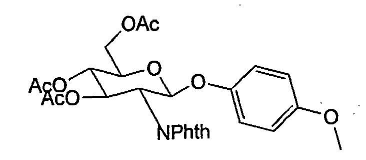

Compound (12) (61 g, 0.13 mol) was dissolved in anhydrous dichloromethane (500 mL) under and argon atmosphere. 4Å molecular sieves and para-methoxyphenol (28.5 g, 0.22 mol) were added. The reaction mixture was cooled to 0°C prior to the addition of boron trifluoride diethyl etherate (20 mL, 0.16 mol). The reaction mixture was warmed to room temperature and stirred for 17 hours after which TLC (1:140-60°C petroleum ether/ethyl acetate) indicated the complete consumption of starting material (R_{f}0.7) and the formation of a single product (R_{f}0.5). The reaction mixture was diluted with 1 L of dichloromethane and washed with water (1 L), a saturated solution of bicarbonate of soda (2 x 1L) and dried over magnesium sulfate before being concentrated *in vacuo.* The crude product loaded onto silica and washed with 3:1 40-60°C petroleum ether/ethyl acetate until all *p*-methoxyphenol was removed. The sample was then eluted using 3 L of ethyl acetate to yield 50.32 g (93 mmol, 71.4% yield) of a white solid. m.p. 138.4 -141.8 °C (ethyl acetate/40-60°C petroleum ether). [α]²⁵_{D} + 61.0 (CHCl₃, c = 1.2) [lit. 46.7 (c = 1.0)]; ¹HNMR (400 MHz, CDCl₃) δ = 1.89, 2.05, 2.11 (3 x s, 3 x 3H,.3 x C(O)OCH₃), 3.73 (s, 3H, OCH₃), 3.94-3.99 (m, 1H, *J*₄₋₅ 9.8 Hz, *J*₅₋₆ 2.3 Hz, *J*_{5-6'} 5.1 Hz, H-5), 4.18 (dd, 1H, *J*_{6-6'} 12.2 Hz, H-6), 4.36 (dd, 1H, H-6'), 4.57 (dd, 1H, *J*₁₋₂ 8.5 Hz, *J*₂₋₃ 10.8 Hz, H-2), 5.26 (dd, 1H, *J*₃₋₄ 9.1 Hz, H-4), 5.86 (dd, 1H-, H-3), 5.86 (d, 1H-H-1), 6.73-6.86 (m; 4H, 4 x Ar-H), 7.74-7.76 (m, 2H, 2 x Ar-H), 7.86-7.88 (m, 2H, 2 x Ar-H). *m*/*z* (ES⁺): 564 (100%, M + Na⁺)

### Example 14: Synthesis of 2-deoxy-1-para-methoxyphenyl-2-phthalimido-β-D-glucopyranoside (14)

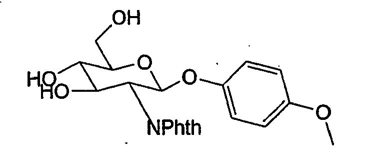

Compound (13) (50.34 g, 93 mmol) was dissolved in 500 mL of anhydrous methanol under an argon atmosphere. Sodium methoxide (89 mg, 1.64 mmol) was added and the reaction was stirred at room temperature for 17 hours after which TLC (1:1 ethyl acetate: 40-60 °C petroleum ether) indicated the complete disappearance of starting material (R_{f} 0.6) and the formation of a single product spot (R_{f} 0.1). The reaction mixture was concentrated *in vacuo* until white crystals started appearing. The crude reaction mixture was then placed at -20°C overnight to yield 33.04 g (80 mmol, 86% yield) of a white crystalline solid. M.p. 220-221°C (methanol). [α]²⁶_{D} + 29.2 (CHCl₃, c = 1.0); ¹H NMR (400 MHz, DMSO) δ = 3.28-3.33 (m, 1H, *J*_{OH-4} 5.7 Hz, H-4), 3.42-3.46 (m, 1H, *J*₅₋₆ 1.1 Hz, H-5), 3.54-3.60 (m, 1H, *J*_{6-6'} 11.6 Hz, H-6'), 3.65 (s, 3H, OCH₃), 3.77 (ddd, 1H, *J*_{OH-6} 5.3 Hz, H-6), 4.01 (dd, 1H, *J*₁₋₂ 8.4 Hz, *J*₂₋₃ 10.3 Hz, H-2), 4.07-4.13 (m, 1H, *J*_{OH-3} 5.3 Hz, H-3), 4.69 (t, 1H, OH-6), 5.27 (d, 1H, OH-4), 5.55 (d, 1H, H-1), 6.76-6.83 (m, 4H, Ar-H), 7.87-7.95 (m, 4H, Ar-H). ¹³C NMR (100.6 MHz, DMSO), 56.17 (OCH₃), 57.97 (C-2), 62.45 (C-6), 71.18 (C-4), 71.57 (C-3), 78.51 (C-5), 97.93 (C-1), 115.42, 118.55, 123.96, 124.26 (CH-Ar), 135.57, 151.61, 155.61 (C-Ar). *m*/*z* (ES⁻): 414 (100%, M-H⁻).

### Example 15: Synthesis of (R)-4,6-O-Benzylidene-2-deoxy-1-para-methoxyphenyl-2-phthalimido-β-D-glucopyranoside (15)

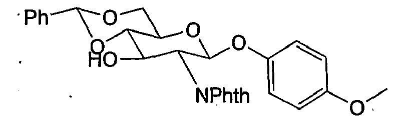

Compound (14) (33 g, 80 mmol) was dissolved in acetonitrile (500 mL). Benzaldehyde dimethyl acetal (25 mL, 167 mmol, 2 equivalents) was added, as was para-toluenesulfonic acid (3.15 g, 16.5 mmol). The reaction mixture was stirred for 14 hours at room temperature under an argon atmosphere when TLC (3:1 toluene: ethyl acetate) indicated the disappearance of starting material (R_{f} 0.0) and the formation of a major product (R_{f} 0.6). Triethylamine (6 mL) was added prior to the mixture being concentrated *in vacuo.* The crude product was recrystallised from hot methanol to yield 34 g (68 mmol, 85% yield) of a white crystalline solid. m.p. 117.9-119.2°C. [α]²⁷_{D} +17.8 (CHCl₃, c = 1.3) [lit. 9.7 (c = 1.0)]; ¹H NMR (400 MHz, CDCl₃) δ = 3.38 (br s, 1H, OH), 3.69-3.77 (m, 2H, *J*₃₋₄ 8.4 Hz, *J*_{5-6'} 4.0 Hz, H-4, H-5), 3.73, (s, 3H, O-CH₃), 3.88 (app t, 1H, *J*₆₋₆, 9.8 Hz, H-6), 4.41 (dd, 1H, H-6'), 4.52 (dd, 1H, *J*₁₋₂ 8.5 Hz, *J*₂₋₃ 10.5 Hz, H-2), 4.72 (dd, 1H, H-3), 5.60 (1H, s, PhCH), 5.81 (d, 1H, H-1), 6.73-6.77 (m, 2H, 2 x Ar-H), 6.84-6.88 (m, 2H, 2 x Ar-H), 7.38-7.40 (m, 2H, 2 x Ar-H), 7.50-7.57 (m, 2H, 3 x Ar-H), 7.73-7.75 (m, 2H, 2 x Ar-H), 7.86-7.90 (m, 2H, 2 x Ar-H)^{[59]}. *m*/*z* (ES⁺): 526 (100%, M + Na⁺).

### Example 16: Synthesis of 6-O-benzyl-2-deoxy-1-para-methoxyphenyl-2-phthalimido-β-D-glucopyranoside (16)

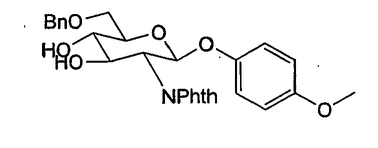

*Method 1:* Compound (15) (434 mg, 0.84 mmol) was dissolved in anhydrous tetrhydrofuran (10 mL). 4 Å powdered molecular sieves were added and the mixture was stirred at room temperature under an argon atmosphere for 30 minutes prior to being cooled to 0°C. Methyl orange (∼ 1 mg) was added to the reaction mixture followed by sodium cyanoborohydride (746 mg, 12 mmol). A solution of hydrogen chloride in 1,4-dioxane (4M) was added until the reaction mixture turned from orange to pink. Over the next 4 hours more hydrogen chloride solution was added to maintain the pink coloration. The reaction was then left for 12 hours after which it was still pink. TLC (3:1 toluene:ethyl acetate) indicated the formation of a product (R_{f} 0.1) and the presence of starting material. An additional portion of sodium cyanoborohydride was added (256 mg, 4.1 mmol) as was more hydrogen chloride solution to maintain a pink coloration to the reaction mixture. A TLC taken 23 hours later indicated the complete consumption of starting material. Ice water (100 mL) was added and the aqueous layer was extracted with dichloromethane (3 x 100 mL). The organic layer was stirred overnight with 1N hydrogen chloride solution in water, prior to being washed with 200 mL of a saturated solution of bicarbonate of soda and 100 mL of a saturated sodium chloride solution. The organic layer was dried over magnesium sulfate and concentrated *in vacuo.* The residue was purified by flash silica chromatography to yield 240.8 mg (43% yield) of a white solid. [α]²¹_{D} +11.8 (CH₃COCH₃, c =1.0); ¹H NMR (400 MHz, DMSO) δ = 3.29-3.35 (m, 1H, *J*_{4-OH} 5.9 Hz, H-4), 3.60-3.70 (m, 2H, H-5, H-6), 3.65 (s, 3H, OCH₃), 3.81-3.86 (m, 1H, H-6'), 4.03 (dd, 1H, *J*₁₋₂ 8.6 Hz, *J*₂₋₃ 10.3 Hz, H-2), 4.09-4.15 (m, 1H, *J*_{OH-3} 4.6 Hz, H-3), 4.53 (d, 1H, ¹*J* 12.1 Hz, CHH'-Ph), 4.56 (d, 1H, CHH'Ph), 5.42 (d, 1H, OH-4), 5.58 (d, 1H, OH-3), 5.599 (d, 1H, H-1), 6.73-6.76 (m, 2H, Ar-H), 6.81-6.84 (m, 2H, Ar-H), 7.26-7.37 (m, 5H, Ar-H), 7.88-7.94 (m, 4H, Ar-H). ¹³C NMR (100.6 MHz, DMSO) 56.16 (OCH₃), 57.91 (C-2), 70.24 (C-6), 71.46, 71.56 (C-3, C-4), 73.03 (CH₂Ph), 76.87 (C-5), 97.80 (C-1), 115.36, 118.70, 123.99, 124.28, 128.15, 129.03, 135.60 (CH-Ar), 139.48, 151.42, 155.66 (C-Ar). *m*/*z* (ES⁺): 564 (M+CH₃CN+NH₄, 100%)

*Method* 2: Compound (15) (4.90 g, 9.4 mmol) was dissolved in anhydrous dichloromethane (100 mL). 4Å powdered molecular sieves were added. Trifluoromethanesulfonic anhydride (15 mL, 108 mmol) and trifluoromethanesulfonic acid (7 mL, 79 mmol) were added and the mixture was stirred at room temperature for 30 minutes prior to being cooled to 0°C. Triethylsilane (10 mL, 63 mmol) was added and the reaction was stirred at 0°C for 2 hours. TLC (3:1 toluene/ethyl acetate) showed only the slightest formation of product, so the reaction mixture was warmed to room temperature and an additional portion of triethylsilane (5 mL, 38 mmol) was added. A TLC taken 3 hours after the addition of this second portion indicated the complete consumption of starting material and the formation of a single product (R_{f} 0.2). Triethylamine (20 mL) was added dropwise over 10 minutes to the reaction mixture. The reaction mixture was then extracted with chloroform (300 mL) and the organic layer washed with a saturated solution of bicarbonate of soda (500 mL), water (300 mL) and a saturated solution of sodium chloride (200 mL) prior to being purified by flash silica chromatography (3:2 40-60°C petroleum ether/ethyl acetate) to yield 3.02 g (5.8 mmol, 62% yield) of a white solid identical to that described above.

### Example 17: Synthesis of N-Acetyl-d-glucosamine-derivatised CLIO particles

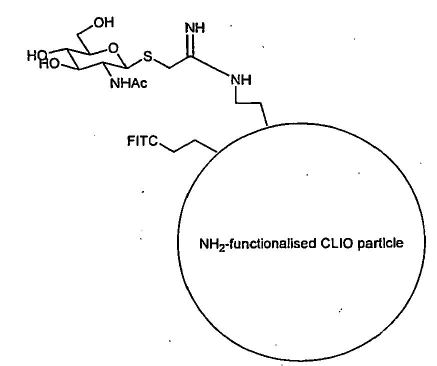

Compound (4) (38.4mg, 0.14mmol) was dissolved in 2ml of dry methanol and placed under argon. Sodium methoxide (7.6mg 0.14mmol) was added and the mixture was reacted for 18h after which the mixture was concentrated in vacuo.

The crude mixture was re-dissolved in sodium tetraborate buffer (0.1M, pH 9.5, 1.0 mL). Colloidal suspension of CLIO-particles (60, 13.8 mg/mL in 0.1 M sodium tetraborate buffer; pH 9.5, 1 mL) was added. The mixture was stirred gently at room temperature for 2 hours prior to purification by dialysis using SpectraPor Cellulose Ester dialysis tubing (12-14 kDa MWCO, 2 L, 3 changes). The product solution was lyophilized and dissolved in 2.0 mL of water for in vivo analysis.

### Example 18: Synthesis of N-Acetyl-d-lactosamine derived CLIO-particles

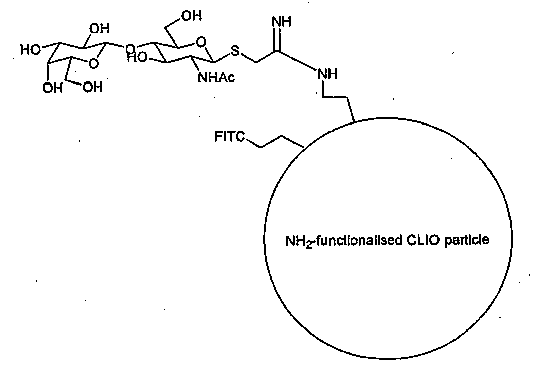

1-Thio-*S*-cyanomethyl-*N*-acetyllactosamine (43.1mg, 0.10mmol) was dissolved in anhydrous methanol (3 mL) and placed under an argon atmosphere. Sodium methoxide (6.8mg 0.13mmol) was added and the mixture was reacted for 18h after which the reaction mixture was concentrated *in vacuo.*

Colloidal suspension of CLIO-particles (60, 13.8 mg/mL in 0.1 M sodium tetraborate buffer, pH 9.5, 2 mL) was added to the residue. The suspension was stirred at room temperature for 2.5 hours prior to purification by Sephadex PD-10 desalting column chromatography. The fraction containing CLIO-particles was lyophilized and redissolved in 1.0 mL and stirred at room temperature with of a saturated solution of fluorescein isothiocyanate in water (2 mL). The particles were purified by vivaspin and redissolved in 1 mL of water.

### Example 19: Synthesis of Lewis^{x}-derivatised CLIO particles

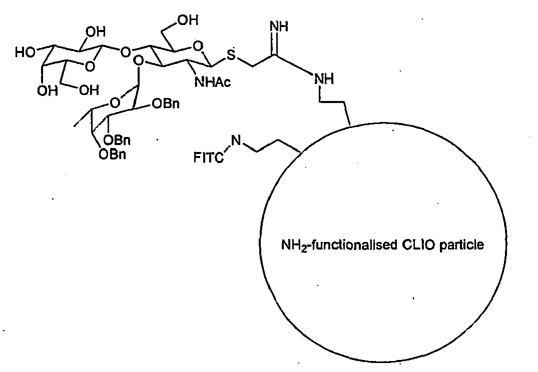

IME reagent (42 mg, 0.05 mmol) was dissolved in a solution of methanolic sodium (0.1 M, 5 mL) and stirred at room temperature for 19 hours. The mixture was concentrated *in vacuo.* A solution of amine-terminated magnetic particle in sodium tetraborate solution (20 mg/mL, 0.1 M, pH 8.8) was added to the resulting solid. The mixture was stirred at room temperature for 3 hours prior to purification by vivaspin column and buffer replacement with water. A saturated solution of fluorescein isothiocyanate (2 mL) was added to the particle solution and stirred at room temperature for 2 h. The particles were again purified by vivaspin column (10,000 MWCO) and redissolved in 2 mL of water).

### Example 20: Synthesis of Sialyl Lewis^{x}-derived magnetic particle

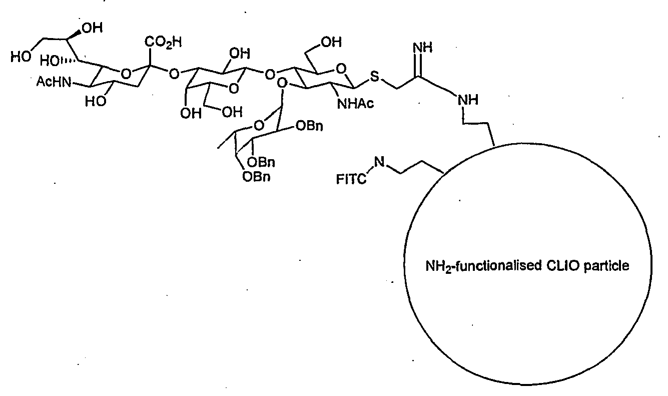

The *N*-acetyllactosamine-derived, FITC-labelled magnetic particle solution was concentrated by vivaspin and redissolved in 2 mL of sodium cacodylate buffer (20 mM, pH 7.5). CMP-Sialic acid (10 mg) and GDP-Fucose (10 mg) were added. 0.01 U of α-2,3-sialyltransferase (purified from rat liver) was added and the mixture was stirred for 2 hours at 37°C prior to the addition of 0.01 U of α-1,3-fucosyltransferase. The mixture was stirred at 37°C for an additional 4 hours prior to purification and buffer replacement by vivaspin (10,000 MWCO).

Analogous processes as have been described in Examples 17 to 20 can be carried out using the intermediate described in Example 16.

### Example 21: Use as Contrast agents

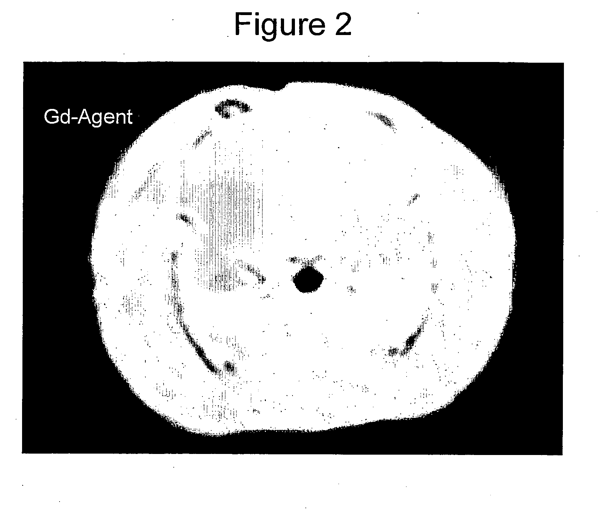

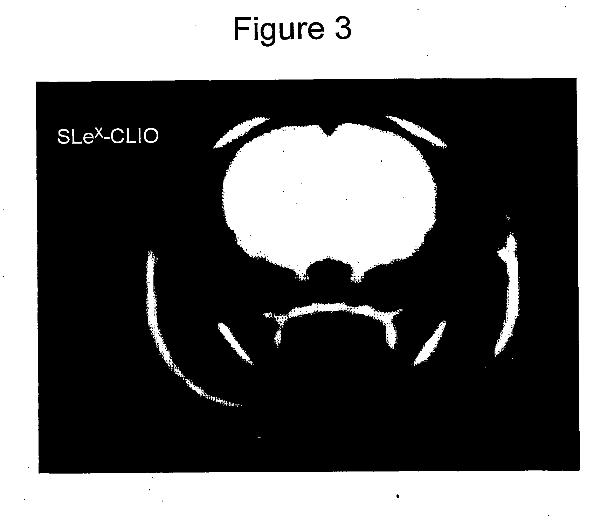

The magnetic particles of the present invention can be used in a number of different methods. In particular, sugars conjugated to ultra small super paramagnetic iron oxide provide more sensitive detection of cerebroendothelial cell activation than know contrast agents such as Gd-DTPA-B(sLEex)A. In particular conjugated magnetic particles in accordance with the present invention were used in MRI scans. Interleukin-1β was injected into the left hemisphere of rat brains. Interleukin beta was used to induce an inflammatory response in the left hemispheres. All samples were subjected to MRI scans. Controls were carried out using Gd alone, or superparamagnetic particles alone. Two different conjugates, one of which having a disaccharide and one of which having Sialyl Lewis X, both in accordance with the present invention were tested. The scan using Sialyl Lewis X is shown in Figure 3. The scans using the conjugates of the present invention showed asymmetry, being darker in the left hemisphere than the right. The control with Gd (shown in Figure 2) confirmed that there was no breakdown of the blood brain barrier. Similarly, for the control with superparamagnetic particles alone no difference was observed between cranial hemispheres. Thus, no asymmetry was seen in either control. Thus, the conjugates of the present invention allowed imaging of the areas of the brain that had been injected with interlukin-1β, and which would be expected to show greater levels of selectin expression.

## Claims

1. A conjugate comprising an iron containing colloidal particle, the particle being conjugated to one or more targeting moieties, wherein the targeting moieties are oligosaccharides, which comprise a saccharide unit selected from GalNAc (N-acetyl galactosamine), GalUA (galacturonic acid), GlcNAc (N-acetyl glucosamine), GlcUA (glucuronic acid), IdUA (iduronic acid) and a sialic acid.

2. A conjugate according to claim 1 wherein the targeting moieties are selected to bind a cell surface receptor, preferably a selectin or lectin receptor, more preferably selectin E and/or selectin P, and/or wherein at least one of the targeting moieties is Sialyl Lewis X.

3. A conjugate according to claim 1 or claim 2 wherein the iron containing colloidal particle is from 5 nm to 1 mm in size, and/or wherein the iron containing colloidal particle comprises iron (II) or iron (III) oxide or an iron hydroxide or a mixture thereof, preferably wherein the iron containing colloidal particle comprises iron (III) oxide, and/or wherein the iron containing colloidal particle is a cross-linked iron oxide particle.

4. A conjugate according to claim 1, wherein the targeting moieties are selected from Sialyl Lewis X (GlcNAc(-Fuc)-Cal-Sia), Lewis X (GlcNAc(-Fuc)-Gal) and GlcNAc-Gal.

5. A formulation comprising a conjugate as claimed in any one of the preceding claims and a pharmaceutically acceptable excipient.

6. A conjugate according to any one of claims 1 to 4 or a formulation according to claim 5 for use in a diagnostic method practised on the human or animal body.

7. Use of a conjugate according to any one of claims 1 to 4 in the manufacture of a formulation for use in medical imaging, preferably wherein the medical imaging technique is magnetic resonance imaging, and/or wherein the conjugate is used to monitor and/or diagnose inflammation or used in the diagnosis of multiple sclerosis.

8. A method for enhancing the contrast of an image obtained by a medical imaging technique, which method comprises, prior to the image being formed, the administration of a conjugate according to any one of claims 1 to 4 or a formulation according to claim 5.

9. A process for preparing an intermediate which comprises:
(a) providing a compound of formula (I):
R¹-S-CH₂CN (I)
wherein R¹ is an acetylated carbohydrate-based moiety;
(b) selectively deprotecting the compound of formula (I) in order to convert the acetylated groups to hydroxy groups;
(c) reacting the product of step (b) with a carbohydrate processing enzyme in order to extend the compound of formula (I) by the addition of one or more carbohydrate-based moieties R²; and
(d) activating the product of step (c) in order to produce a compound of formula (II):
(R²)ₙR¹-S-CH₂-C(NH)-O-Mc (II)
wherein n is the number of carbohydrate-based moieties R² added in step (c) and is an integer of from 1 to 10.

10. A process as claimed in claim 9 wherein R¹ is saccharide wherein the hydroxy groups -OH have been protected as -OAe groups, preferably wherein R¹ is a protected monosaccharide, more preferably wherein R¹ is a protected glucosamine, and/or
wherein the carbohydrate processing enzyme extends R¹ by the addition of one or more further carbohydrate-based moieties R², and/or wherein the carbohydrate processing enzyme is selected from galactosyltransferase, α-2,3-sialyltransferase and α-1,3-fucosyltransferasc.

11. A process as claimed in claim 9 or claim 10 wherein n is from 1 to 6, more preferably from 1 to 4, more preferably 1 or 2, and is most preferably 1.

12. A disaccharide of formula (III):
R²-R¹-S-CH₂-C(NH)-O-Me (III)
wherein R¹ and R² are monosaccharide moieties.

13. A process for preparing a conjugate, which conjugate comprises an iron containing colloidal particle, the particle being conjugated to one or more targeting moieties, wherein the targeting moieties arc oligosaccharides, which process comprises reacting an intermediate of formula (II) as produced by any one of claims 9 to 11 or an intermediate of formula (III) as defined in claim 12 with an iron containing colloidal particle.

14. A process as claimed in claim 13, which further comprises reacting the product with a carbohydrate processing enzyme, which enzyme is preferably selected from α-2,3-sialyltransferase and α-1,3-fucosyltransferase.

15. A process for preparing a compound of formula (I), which compound is as defined in claim 9, which process comprises:
(a) providing a compound of formula (IV):
R¹-O-C₆H₄-OR³ (IV)
wherein R³ is a C₁₋₆ alkyl group and R¹ is as defined in claim 9;
(b) reacting the compound of formula (IV) with ammonium cerium nitrate to produce a compound of formula (V):
R¹-OH (V)
(c) reacting the compound of formula (V) with a thionyl halide, preferably thionyl chloride, and subsequently reacting with thiourea to produce a compound of formula (VI):
R¹-S-C(=NH)-NH₂ (VI)
(d) reacting the compound of formula (VI) with chloroacetonitrile under conditions sufficient to produce a compound of formula (I).

## Patentansprüche

1. Konjugat, umfassend ein eisenhaltiges kolloidales Teilchen, wobei das Teilchen an einem oder mehreren zielsuchenden Resten konjugiert ist, wobei die zielsuchenden Reste Oligosaccharide sind, welche eine Saccharideinheit umfassen, die aus GalNAc (N-Acetylgalactosamin), GalUA (Galacturonsäure), GlcNAc (N-Acetylglucosamin), GlcUA (Glucuronsäure), IdUA (Iduronsäure) und einer Sialsäure gewählt ist.

2. Konjugat gemäß Anspruch 1, wobei die zielsuchenden Reste so gewählt sind, um einen Zelloberflächenrezeptor zu binden, vorzugsweise einen Selektin- oder Lektinrezeptor, stärker bevorzugt Selektin E und/oder Selektin P, und/oder wobei mindestens einer der zielsuchenden Reste Sialyl-Lewis X ist.

3. Konjugat gemäß Anspruch 1 oder Anspruch 2, wobei das eisenhaltige kolloidale Teilchen 5 nm bis 1 mm groß ist und/oder wobei das eisenhaltige kolloidale Teilchen Eisen(II)- oder Eisen(III)-oxid oder ein Eisenhydroxid oder eine Mischung davon umfasst, wobei vorzugsweise das eisenhaltige kolloidale Teilchen Eisen(III)-oxid umfasst und/oder wobei das eisenhaltige kolloidale Teilchen ein vernetztes Eisenoxidteilchen ist.

4. Konjugat gemäß Anspruch 1, wobei die zielsuchenden Reste aus Sialyl-Lewis X (GlcNAc)(-Fuc)-Gal-Sia), Lewis X (GlcNAc(-Fuc)-Gal) und GlcNAc-Gal gewählt werden.

5. Formulierung, umfassend ein Konjugat gemäß einem Beliebigen der vorhergehenden Ansprüche und einen pharmazeutisch annehmbaren Arzneimittelträger.

6. Konjugat gemäß einem Beliebigen der Ansprüche 1 bis 4 oder eine Formulierung gemäß Anspruch 5 für die Verwendung in einem Diagnoseverfahren, das am menschlichen oder tierischen Körper praktiziert wird.

7. Verwendung eines Konjugats gemäß einem Beliebigen der Ansprüche 1 bis 4 bei der Herstellung einer Formulierung für die Verwendung in der medizinischen Bildgebung, wobei vorzugsweise die medizinische Bildgebungstechnik eine Bildgebung mittels magnetischer Resonanz ist und/oder wobei das Konjugat zur Überwachung und/oder Diagnose von Entzündungen verwendet wird oder bei der Diagnose von multipler Sklerose verwendet wird.

8. Verfahren zum Verstärken des Kontrasts eines Bildes, das durch eine medizinische Bildgebungstechnik erhalten wird, wobei das Verfahren, bevor das Bild gebildet wird, die Verabreichung eines Konjugats gemäß einem Beliebigen der Ansprüche 1 bis 4 oder einer Formulierung gemäß Anspruch 5 umfasst.

9. Verfahren für die Herstellung eines Zwischenprodukts, welches folgendes umfasst:
(a) Bereitstellen einer Verbindung der Formel (I):
R¹-S-CH₂CN (I)
worin R¹ ein acetylierter Rest auf Kohlenhydratbasis ist;
(b) selektives Entschützen der Verbindung der Formel (I), um die acetylierten Gruppen zu Hydroxygruppen umzuwandeln;
(c) Umsetzen des Produkts von Schritt (b) mit einem Kohlenhydrat prozessierenden Enzym, um die Verbindung der Formel (I) durch die Hinzufügung von einem oder mehreren Resten R² auf Kohlenhydratbasis zu erweitern; und
(d) Aktivieren des Produkts von Schritt (c), um eine Verbindung der Formel (II) herzustellen:
(R²)ₙ-R¹-S-CH₂-C(NH)-O-Mc (II)
worin n die Zahl der Reste R² auf Kohlenhydratbasis ist, die im Schritt (c) zugefügt werden, und eine ganze Zahl von 1 bis 10 ist.

10. Verfahren gemäß Anspruch 9, wobei R¹ Saccharid ist, worin die Hydroxygruppen -OH als -OAc-Gruppen geschützt wurden, wobei vorzugsweise R¹ ein geschütztes Monosaccharid ist, wobei stärker bevorzugt R¹ ein geschütztes Glucosamin ist und/oder wobei das Kohlenhydrat prozessierende Enzym R¹ durch die Hinzufügung von einem oder mehreren weiteren Resten R² auf Kohlenhydratbasis erweitert und/oder wobei das Kohlenhydrat prozessierende Enzym aus Galactosyltransferase, α-2,3-Sialyltransferase und α-1,3-Fucosyltransferase gewählt ist.

11. Verfahren gemäß Anspruch 9 oder Anspruch 10, wobei n 1 bis 6, stärker bevorzugt 1 bis 4, noch stärker bevorzugt 1 oder 2, und am meisten bevorzugt 1 ist.

12. Disaccharid der Formel (III):
R²-E¹-S-CH₂-C(NH)-O-Me (III)
worin R¹ und R² Monosaccharidreste sind.

13. Verfahren für die Herstellung eines Konjugats, wobei das Konjugat ein eisenhaltiges kolloidales Teilchen umfasst, wobei das Teilchen an einem oder mehreren zielsuchenden Resten konjugiert ist, wobei die zielsuchenden Reste Oligosaccharide sind, wobei das Verfahren das Umsetzen eines Intermediats der Formel (II), wie durch einen Beliebigen der Ansprüche 9 bis 11 hergestellt, oder eines Intermediats der Formel (III), wie in Anspruch 12 definiert, mit einem eisenhaltigen kolloidalen Teilchen umfasst.

14. Verfahren gemäß Anspruch 13, weiterhin umfassend das Umsetzen des Produkts mit einem Kohlenhydrat prozessierenden Enzym, wobei das Enzym vorzugsweise aus α-2,3-Sialyltransferase und α-1,3-Fucosyltransferase gewählt wird.

15. Verfahren für die Herstellung einer Verbindung der Formel (I), welche Verbindung wie in Anspruch 9 definiert ist, wobei das Verfahren Folgendes umfasst:
(a) Bereitstellen einer Verbindung der Formel (IV):
R¹-O-C₆H₄-OR³ (IV)
worin R³ eine C₁₋₆-Alkylgruppe ist und R¹ wie in Anspruch 9 definiert ist;
(b) Umsetzen der Verbindung der Formel (IV) mit Ammoniumcernitrat zur Herstellung einer Verbindung der Formel (V):
R¹-OH (V)
(c) Umsetzen der Verbindung der Formel (V) mit einem Thionylhalogenid, vorzugsweise Thionylchlorid, und anschließendes Umsetzen mit Thioharnstoff zur Herstellung einer Verbindung der Formel (VI):
R¹-S-C(=NH)-NH₂ (VI)
(d) Umsetzen der Verbindung der Formel (VI) mit Chloracetonitril unter Bedingungen, die ausreichend sind, um eine Verbindung der Formel (I) herzustellen.

## Revendications

1. Conjugué comprenant une particule colloïdale contenant du fer, la particule étant conjuguée à un ou plusieurs groupement de ciblage, dans lequel les groupements de ciblage sont des oligosaccharides, qui comprennent une unité saccharidique choisie parmi GalNAc (N-acétylgalactosamine), GalUA (acide galacturonique), GlcNAc (N-acétylglucosamine), GlcUA (acide glucuronique), IdUA (acide iduronique) et un acide sialique.

2. Conjugué selon la revendication 1 dans lequel les groupements de ciblage sont choisis pour se lier à un récepteur de surface cellulaire, de préférence un récepteur de sélectine ou de lectine, plus avantageusement une sélectine E et/ou une sélectine P, et/ou dans lequel au moins un des groupements de ciblage est le Sialyl Lewis X.

3. Conjugué selon la revendication 1 ou la revendication 2 dans lequel la particule colloïdale contenant du fer a une taille de 5 nm à 1 mm, et/ou dans lequel la particule colloïdale contenant du fer comprend un oxyde de fer (II) ou de fer (III) ou un hydroxyde de fer ou un mélange de ceux-ci, de préférence dans lequel la particule colloïdale contenant du fer comprend un oxyde de fer (III), et/ou
dans lequel la particule colloïdale contenant du fer est une particule d'oxyde de fer réticulée.

4. Conjugué selon la revendication 1, dans lequel les groupements de ciblage sont choisis parmi le Sialyl Lewis X (GlcNAc(-Fuc)-Gal-Sia), Lewis X (GlcNac(-Fuc)-Gal) et GlcNac-Gal.

5. Formulation comprenant un conjugué tel que revendiqué dans l'une quelconque des revendications précédentes et un excipient pharmaceutiquement acceptable.

6. Conjugué selon l'une quelconque des revendications 1 à 4 ou formulation selon la revendication 5 utilisable dans une méthode de diagnostic pratiquée sur le corps humain ou animal.

7. Utilisation d'un conjugué selon l'une quelconque des revendications 1 à 4 dans la préparation d'une formulation utilisable en imagerie médicale, de préférence dans laquelle la technique d'imagerie médicale est l'imagerie par résonance magnétique, et/ou dans laquelle le conjugué est utilisé pour suivre et/ou diagnostiquer une inflammation ou utilisé dans le diagnostic de la sclérose en plaques.

8. Méthode pour stimuler le contraste d'une image obtenue par une technique d'imagerie médicale, méthode qui comprend, avant que l'image ne soit formée, l'administration d'un conjugué selon l'une quelconque des revendications 1 à 4 ou d'une formulation selon la revendication 5.

9. Procédé pour préparer un intermédiaire qui comprend
(a) la fourniture d'un composé de formule (1):
· R¹-S-CH₂CH (I)
dans lequel R¹ est un groupement glucidique acétylé ;
(b) la déprotection sélective du composé de formule (I) afin de convertir les groupes acétylés en groupes hydroxy ;
(c) la réaction du produit de l'étape (b) avec une enzyme de maturation glucidique afin d'allonger le composé de formule (I) par addition d'une ou plusieurs fractions glucidiques R² ; et
(d) l'activation du produit de l'étape (c) afin de produire un composé de formule (II) :
(R²)ₙ-R¹-S-CH₂-C(NH)-O-Mc (II)
dans laquelle n est le nombre de groupements glucidiques R² ajoutés à l'étape (c) et est un nombre entier de 1 à 10.

10. Procédé tel que revendiqué à la revendication 9
dans lequel R¹ est un saccharide dans lequel les groupes hydroxy -OH ont été protégés sous forme de groupes -OAc, de préférence dans lequel R¹ est un monosaccharide protégé, plus avantageusement dans lequel R¹ est une glucosamine protégée, et/ou dans lequel l'enzyme de maturation glucidique allonge R¹ par addition d'une ou plusieurs autres fractions glucidiques R², et/ou dans lequel l'enzyme de maturation glucidique est choisie parmi la galactosyltransférase, l'a-2,3-sialyltransférase et l'α-1,3-fucosyltransférase.

11. Procédé tel que revendiqué à la revendication 9 ou la revendication 10, dans lequel n est 1 à 6, plus avantageusement 1 à 4, plus avantageusement 1 ou 2, et le plus avantageusement 1.

12. Disaccharide de formule (III) :
R²-R¹-S-CH₂-C(NH)-O-Me (III)
dans laquelle R¹ et R² sont des groupements monosaccharidiques.

13. Procédé de préparation d'un conjugué, conjugué qui comprend une particule colloïdale contenant du fer, la particule étant conjuguée à un ou plusieurs groupements de ciblage, dans lequel les groupements de ciblage sont des oligosaccharides, procédé qui comprend la réaction d'un intermédiaire de formule (II) tel que produit par l'une quelconque des revendications 9 à 11 ou un intermédiaire de formule (III) tel que défini à la revendication 12 avec une particule colloïdale contenant du fer.

14. Procédé tel que revendiqué à la revendication 13, qui comprend en outre la réaction du produit avec une enzyme de maturation glucidique, enzyme qui est de préférence choisie parmi l'α-2,3-sialyltransférase et l'α-1,3-fucosyltransférase.

15. Procédé de préparation d'un composé de formule (I), composé qui est tel que défini à la revendication 9, procédé qui comprend :
(a) la fourniture d'un composé de formule (IV) :
R¹-O-C₆H₄-OR³ (IV)
dans laquelle R³ est un groupe alkyle en C₁-C₆ et R¹ est tel que défini à la revendication 9 ;
(b) la réaction'du composé de formule (IV) avec le nitrate d'ammonium et de cérium pour produire un composé de formule (V) ;
R¹- OH (V)
(c) la réaction du composé de formule (V) avec un halogénure de thionyle, de préférérence un chlorure de thionyle, et ensuite la réaction avec de la thio-urée pour produire un composé de formule (VI) :
R¹-S-C(=NH)-NH₂ (VI)
(d) la réaction du composé de formule (VI) avec le chloro-acétonitrile dans des conditions suffisantes pour produire un composé de formule (I).
